Europäisches Patentamt

European Patent Office

Office européen des brevets

(19)

(11) Veröffentlichungsnummer: **0 290 375**
**A1**

(12) # EUROPÄISCHE PATENTANMELDUNG

(21) Anmeldenummer: 88730098.6

(22) Anmeldetag: 27.04.88

(51) Int. Cl.⁴: **A 61 F 2/46**
G 01 H 17/00, B 25 D 13/00,
B 25 D 1/00

(30) Priorität: 01.05.87 DE 3714966

(43) Veröffentlichungstag der Anmeldung:
09.11.88 Patentblatt 88/45

(84) Benannte Vertragsstaaten: **CH DE FR GB LI**

(71) Anmelder: **MECRON medizinische Produkte GmbH**
**Nunsdorfer Ring 23-29**
**D-1000 Berlin 48 (DE)**

(72) Erfinder: **Buhr, Michael, Dr.**
**Endenicher Allee 32**
**D-5300 Bonn (DE)**

(74) Vertreter: **Christiansen, Henning, Dipl.-Ing.**
**Patentanwalt Pacelliallee 43/45**
**D-1000 Berlin 33 (DE)**

(54) **Vorrichtung zur messtechnischen Kontrolle des Einschlagens einer zementfrei implantierbaren Schaftprothese.**

(57) Vorrichtung zum Messen der kraftschlüssigen Verbindung einer zementfrei implantierbaren Schaftprothese (1) mit einem Knochenschaft, in den die Schaftprothese (1) mittels einer Einschlagvorrichtung (2, 3, 5, 6) eintreibbar ist, mit einem Sensor (4; 54, 64) zur Erfassung der Dämpfung bzw. Abbremsverzögerung der von der Einschlagvorrichtung (2, 3, 5, 6) auf die Schaftprothese (1) ausgeübten Schlagimpulse und einer Signalspeicher- und Auswerteinrichtung (7, 8), die ein vom Sensor (4, 54, 64) abgegebenes Impulsdämpfungssignal (b) als Maß für die kraftschlüssige Verbindung zwischen Schaftprothese (1) und Knochenschaft proportionales Meßsignal abgibt.

F I G. 5

4, 54, 64    7    8    9

EP 0 290 375 A1

Bundesdruckerei Berlin

## Beschreibung

**Vorrichtung zur meßtechnischen Kontrolle des Einschlagens einer zementfrei implantierbaren Schaftprothese**

Die Erfindung betrifft eine Vorrichtung der im Oberbegriff des Anspruchs 1 angegebenen Art.

Im Gegensatz zur zementierten Prothese, die formschlüssig im Oberschenkelschaft verankert wird, wird bei der Im plantation zementloser Prothesenschäfte eine kraftschlüssige Verbindung zwischen Prothesenschaft und Markhöhle des betreffenden Knochens durch das Eintreiben des Prothesenschaftes in den Knochen erzeugt. Dabei beruht die Primärfestigkeit zwischen Prothesenschaft und Knochen ausschließlich auf dem manuellen Geschick und der individuellen operativen Erfahrung des Chirurgen. Bei zu großer Krafteinwirkung auf die Einschlagvorrichtung zum Eintreiben der Schaftprothese in die Markhöhle des Knochens kann es zu unerwünschten Fissuren bzw. Frakturen des Knochenschaftes kommen, wodurch einerseits postoperative Schmerzen hervorgerufen werden können und andererseits die Festigkeit der Verbindung zwischen Prothesenschaft und Knochenschaft erheblich herabgesetzt wird.

Aufgabe der vorliegenden Erfindung ist es, eine Vorrichtung zum meßtechnichen Erfassen des Kraftschlusses der Verbindung zwischen zementfrei implantierter Prothese und Knochenschaft zu schaffen, mit der objektiv angegeben werden kann, wann der günstigste Sitz des Prothesenschaftes mit ausreichendem Sicherheitsabstand zur Fraktur bzw. Fissur des Knochenschaftes erreicht ist. Diese Aufgabe wird durch die kennzeichnenden Merkmale im Anspruch 1 gelöst.

Die erfindungsgemäße Lösung ermöglicht eine objektive Angabe über die optimalen Festigkeit des Sitzes des Prothesenschaftes unter Berücksichtigung eines ausreichenden Sicherheitsabstandes zum möglichen beim Fortsetzen des Einschlagens Auftreten einer Fissur bzw. Fraktur des mit der Prothese versehenen Knochenschaftes.

Die erfindungsgemäße Lösung geht von der theoretischen Überlegung aus, daß die Dämpfung bzw. Abbremsverzögerung einer auf die Schaftprothese auftreffenden, impulsförmigen Kraft sich proportional zur Festigkeit des Implantatsitzes verhält, so daß eine Messung der Dämpfung bzw. der Abbremsverzögerung ein Kriterium für die Festigkeit des Sitzes der Schaftprothese im Knochenschaft darstellt, wobei mit zunehmendem Dämpfungsgrad die Festigkeit des Sitzes wächst. Wird ein bestimmter Dämpfungsgrad überschritten, so besteht die Gefahr einer Fraktur bzw. Fissur, so daß aufgrund von Versuchen Werte des Dämpfungsgrades angebbar sind, bei denen mit ausreichendem Sicherheitsabstand keine Fraktur bzw. Fissur zu erwarten ist.

Eine vorteilhafte Ausgestaltung der erfindungsgemäßen Lösung ist dadurch gekennzeichnet, daß der Sensor im Verlauf der Schlagachse eines Einschlaghammers bzw. eines Einschlaginstruments angeordnet ist bzw. daß der Sensor in einem Testgerät vorgesehen ist, das einen mechanisch oder elektromagnetisch angetriebenen Stößel, eine Auslöseeinrichtung und den die Beschleunigung bzw. Dämpfung des Stößels erfassenden Sensor aufweist.

Durch die Anordnung des Sensors im Verlauf der Schlagachse der Einschlagvorrichtung kann während des Eintreibens der Schaftprothese in die Markhöhle des prothesenschaftaufnehmenden Knochens die Impulsdämpfung erfaßt werden, so daß der Operateur beim Erreichen einer bestimmten, vorgegebenen Impulsdämpfung weiß, daß eine optimale Festigkeit zwischen Prothesenschaft und schaftaufnehmendem Knochen erreicht ist. Mit Hilfe eines Testge rätes, das wahlweise eine fest vorgegebene Schlagkraft auf die Schaftprothese ausüben kann, besteht die Möglichkeit, unabhängig von der Einschlagvorrichtung die Festigkeit des Sitzes zwischen Schaftprothese und schaftaufnehmendem Knochen zu überprüfen, um gegebenenfalls die Prothese tiefer in den schaftaufnehmenden Knochen einzutreiben.

Der Sensor kann wahlweise aus einer Meßspule mit induktiver Erfassung der Impulsdämpfung oder einem Beschleunigungsaufnehmer, vorzugsweise einem piezoelektrischem Wandler, bestehen.

Eine weitere vorteilhafte Ausgestaltung der erfindungsgemäßen Lösung ist dadurch gekennzeichnet, daß die Signalspeichereinrichtung den zeitlichen Verlauf des Impulsdämpfungssignals, die der Beschleunigung bzw. Dämpfung oder Abbremsverzögerung der Schaftprothese entsprechen, registriert und daß die Auswerteinrichtung den Dämpfungsgrad des Impulsdämpfungssignals ermittelt und dem Dämpfungsgrad proportionale Meßsignale an eine Anzeigeeinrichtung abgibt.

Mit Hilfe der Signalspeichereinrichtung kann der Verlauf des Impulsdämpfungssignals erfaßt und gespeichert werden und durch eine geeignete Auswertung des Signalverlaufs auf die Dämpfung und damit die Festigkeit des Sitzes geschlossen werden. Da es sich beim Signalverlauf um eine gedämpfte Schwingung beliebiger Art handeln kann, können verschiedene Methoden zur Bestimmung der Dämpfung grades herangezogen werden. So besteht die Möglichkeit, die Dämpfung aus der Zeit zwischen Auftreffen des Schlags und dem (ersten) Nulldurchgang der gedämpften Schwingung und/oder einemerstenNulldurchgangundeinemfolgendenNulldurchgangder gedämpften Schwingung zu ermitteln. Eine weitere Möglichkeit besteht darin, die Dämpfung aus der Neigung der ersten Schwingung nach Erreichen des Beschleunigungsmaximums oder der Neigung des Mittelwerts der Schwingungen abzuleiten. Eine weitere Möglichkeit besteht darin, die Zeit vom Aufschlag bis zu einem Zeitpunkt zu erfassen, bei dem die Schwingungsamplituden einen vorgegebenen Minimalwert unterschreiten.

In einer bevorzugten Ausführungsform besteht die Auswerteinrichtung aus einem Prozessor, der aus dem gespeicherten Signalverlauf des Impulsdämpfungssignals die Dämpfung ermittelt und in Verbindung mit einem Festspeicher das Kriterium des optimalen Sitzes errechnet, um beim Erreichen dieses optimalen Festigkeitswertes ein entsprechendes akustisches und/oder optisches Signal auszulösen.

Anhand eines in der Zeichnung dargestellten Ausführungsbeispieles soll der der Erfindung zugrunde liegende Gedanke näher erläutert werden.

Es zeigen:

Figur 1 eine schematische Darstellung einer Schaftprothese und einer Einschlagvorrichtung mit an unterschiedlichen Stellen angeordnetem Sensor;

Figur 2 einen Querschnitt durch ein erstes Testgerät mit federmechanisch angetriebenem Stößel und einer Meßspule als Sensor;

Figur 3 einen Querschnitt durch ein zweites Testgerät mit elektromagnetisch angetriebenem Stößel und einem piezoelektrischem Wandler als Sensor;

Figur 4 den zeitlichen Verlauf eines charakteristischen Beschleunigungs- bzw. Dämpfungssignals beim Eintreiben einer Schaftprothese in einen schaftaufnehmenden Knochen;

Figur 5 ein Blockschaltbild der Vorrichtung zum Messen der Festigkeit der kraftschlüssigen Verbindung;

Figur 6 ein Blockschaltbild der Vorrichtung gemäß Figur 5 mit einem Mikroprozessor als Auswerteinrichtung;

Figur 7 ein vereinfachtes Blockschaltbild zur Bestimmung der Zeit zwischen Aufschlag und erstem Nulldurchgang der gedämpften Schwingung und

Figur 8 ein Blockschaltbild zur Bestimmung des Nulldurchgangs des Mittelwertes der gedämpften Schwingung sowie Auslösung einer optischen und/oder akustischen Anzeige.

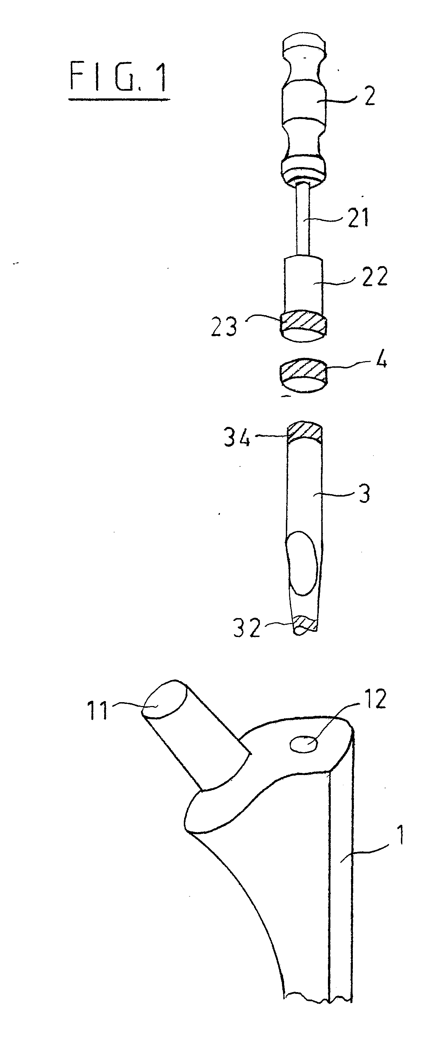

Die in Figur 1 dargestellte Anordnung zeigt schematisch eine zementfrei zu implantierende Femur-Schaftprothese I, die im Bereich des Prothesenkopfes einen konischen Zapfen 11 zur Aufnahme eines Kugelgelenks und eine Einkerbung 12 zum Einsetzen eines Einschlaginstrumentes oder Impactors 3 aufweist.

Üblicherweise besteht die Einschlagvorrichtung aus dem genannten Einschlaginstrument bzw. Impactor 3 und einem Ein schlaghammer 2, der ein Gewicht 22 aufweist, das auf einer Stange 21 gleitend angeordnet ist und mit definierter Kraft auf einen Hammerkopf 23 auftrifft.

In der dargestellten Anordnung kann der Sensor 4 zur Erfassung des Impulsdämpfungssignal im Bereich des Hammerkopfes 23, im Kopf 31 des Einschlaginstruments oder Impactors 3 oder in dessen in die Einkerbung 12 einsetzbaren Fuß 32 vorgesehen werden. Eine weitere Möglichkeit besteht darin, den Sensor 4 getrennt vorzusehen und im Wege der Schlagachse der Einschlagvorrichtung 2, 3 anzuordnen.

Die Ausgangssignale des Sensors 4 werden an eine getrennt angeordnete Signalspeicher- und Auswerteinrichtung abgegeben, die in Verbindung mit einer Anzeigevorrichtung dem Operateur Aufschluß über die Dämpfung und damit die Festigkeit der Verbindung zwischen Prothesenschaft und schaftaufnehmendem Knochen gibt.

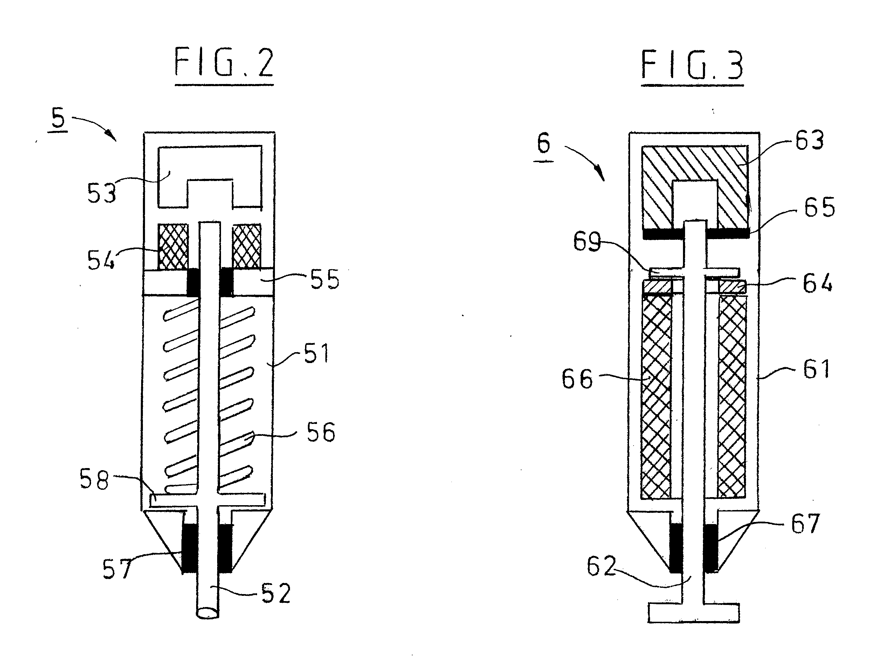

In den Figuren 2 und 3 sind zwei Ausführungsbeispiele für ein Testgerät dargestellt, das entweder als Einschlaghammer (Figur 3) oder als gesondertes Gerät zur Festigkeitsermittlung (Figur 2) eingesetzt werden kann.

Das in Figur 2 dargestellte Testgerät 5 weist ein Gehäuse 51 auf, in dem ein Stößel 52 in mit dem Gehäuse 51 verbundenen Lagern 55, 57 in axialer Richtung gelagert ist. In dem Gehäuse 51 des Testgerätes 5 ist zwischen einem Stößelteller 58 und dem oberen Lager 55 eine Druckfeder 56 als Antriebselement sowie eine mechanische oder elektromagnetische Auslöseeinrichtung 53 vorgesehen, die den Stößel 52 bei gespannter Druckfeder 56 arretiert und auf einen Auslöseimpuls des Operateurs freigibt.

Mittels einer den Stößel 52 umgebenden Meßspule 54 als Sensor kann nach Freigabe des Stößels 52 der Verlauf des Dämpfungssignals erfaßt und an die Signalspeicher- und Auswerteinrichtung weitergeleitet werden. Das in Figur 2 dargestellte Testgerät 5 mit mechanischem Stößelantrieb dient insbesondere als reines Testgerät zur Bestimmung der Festigkeit des Sitzes zwischen Schaftprothese und Knochen, nachdem die Prothese nach Gefühl des Operateurs in den Knochen eingetrieben wurde.

Das in Figur 3 dargestellte Testgerät 6 eignet sich insbesondere zur Verwendung als Einschlaghammer gemäß Figur 1, wobei der Antrieb des Stößels 62 elektromagnetisch mittels einer Antriebsspule 66 erfolgt. Die Erregung der Antriebsspule 66 kann wahlweise in der Weise gesteuert und geregelt werden, daß der Stößel 62 mit definierter, konstanter Geschwindigkeit und Kraft auf das Einschlaginstrument bzw. den Impactor 3 gemäß Figur 1 auftrifft, jedoch ist für die Messung der Festigkeit des Sitzes eine solche definierte, konstante Kraft nicht erforderlich.

In dem Gehäuse 61 des Testgerätes 6 gemäß Figur 3 ist analog zum Testgerät 5 gemäß Figur 2 ein oberes und unteres Lager 65, 67 vorgesehen sowie ein piezoelektrischer Wandler 64, der zwischen der Oberseite des oberen Lagers 65 und einem Stößelteller 69 angeordnet ist und als Beschleunigungsaufnehmer zur Ermittlung des Verlaufs des Dämpfungssignals nach dem Auftreffen auf das Einschlaginstrument dient. Eine Auslöseeinrichtung 63 dient zur Auslösung des Stößels 62 und kann zusätzlich eine Meßwicklung enthalten, die Steuersignale an eine nicht näher dargestellte Steuerelektronik für die Erregung der Antriebsspule 66 abgibt.

Das aus dem Gehäuse 51 bzw. 61 der Testgeräte 5, 6 herausragende Ende des Stößels 52 bzw. 62 kann wahlweise mit einem Hammerkopf oder einer Epitze zum Einsetzen in die Einkerbung 12 gemäß Figur 1 versehen werden.

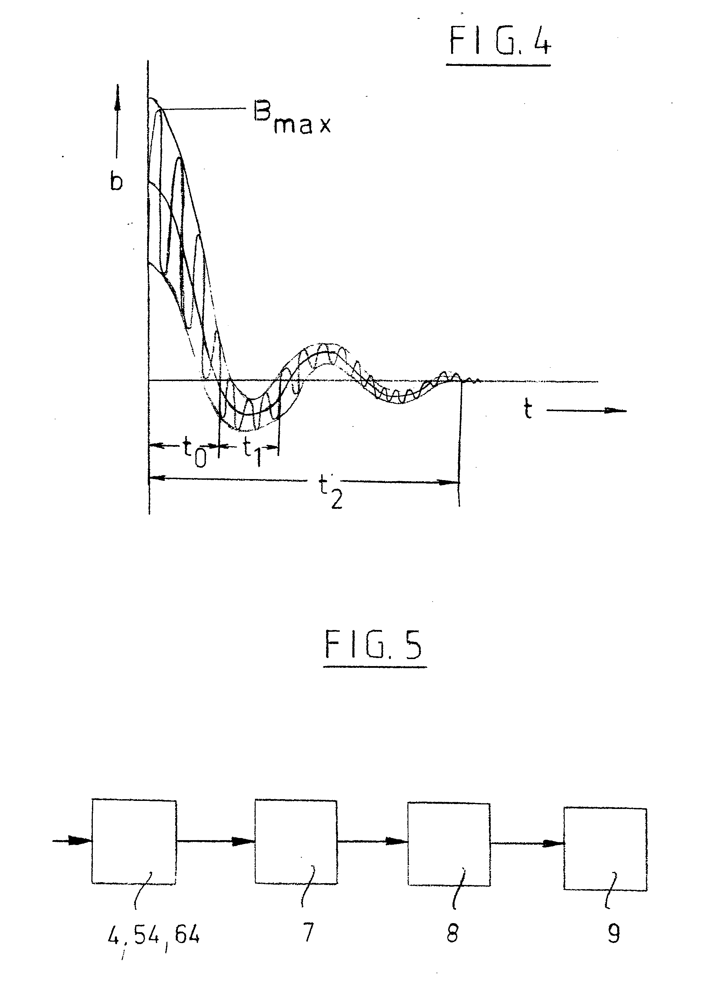

Zur Erläuterung des Funktionsprinzips der erfindungsgemäßen Vorrichtung ist in Figur 4 der typische Verlauf eines Beschleunigungs- bzw. Dämpfungssignals nach dem Auftrefen der Einschlagvorrichtung auf eine Schaftprothese beim bzw. nach dem Eintreiben der Schaftprothese in einen schaftaufnehmenden Knochen dargestellt.

Nachdem Auftreffen der Einschlagvorrichtung auf die Schaftprothese zeigt das Beschleunigungs- bzw. Impulsdämpfungssignal (b) einen Anstieg bis

zu einem Beschleunigungsmaximum (B$_{max}$), woraufhin das Impulsdämpfungssignal (b) nach Art einer gedämpften Schwingung mit einer Vielzahl von Schwingungen bis zur Nullachse abfällt bzw. in die Nullachse einschwingt. Zur Ermittlung der Dämpfung grades können verschiedene Methoden zur Auswertung des Signalverlaufs angewandt werden:

a) Messen der Zeitspanne t$_0$ zwischen Auftreffen der Einschlagvorrichtung auf die Schaftprothese und dem ersten Nulldurchgang der Schwingungen des Impulsdämpfungssignals;

b) Messen der Zeitspanne t$_1$ zwischen aufeinanderfolgenden Nulldurchgängen;

c) Ermitteln der Kurvenneigung des Mittelwerts der Schwingungen des Impulsdämpfungssignals;

d) Ermitteln der Zeitspanne t$_2$ vom Auftreffen der Einschlagvorrichtung auf die Schaftprothese bis zum Unterschreiten einer minimalen, vorgegebenen Amplitude der Schwingungen des Impulsdämpfungssignals, d.h. bei Erreichen eines vorgegebenen, minimalen Abstandes der Hüllkurve bzw. Envelope des Impulsdämpfungssignals.

Daneben sind auch andere Meß- oder Berechnungsmethoden möglich, die hier jedoch zum grundsätzlichen Verständnis der Funktionsweise der erfindungsgemäßen Vorrichtung nicht erläutert werden sollen.

Der in Figur 4 dargestellte Verlauf des vom Sensor 4 bzw. 54, 64 abgegebenen Impulsdämpfungssignals (b) wird gemäß dem in Figur 5 dargestellten prinzipiellen Blockschaltbild in einem Signalspeicher 7 abgespeichert und von einer nachgeschalteten Auswerteinrichtung abgetastet, wobei die Auswerteinrichtung 8 die abgetasteten Werte nach den vorstehend beschriebenen Meß- bzw. Berechnungsmethoden zur Ermittlung der Dämpfung der Schwingung auswertet.

Als Signalspeicher 7 eignet sich beispielsweise ein Transientenrecorder, während die Auswerteinrichtung 8 als digitale und/oder analoge Schaltung aufgebaut sein kann. Der Ausgang der Auswerteinrichtung 8 ist mit einer geeigneten Anzeigeeinrichtung 9 verbunden, die den Verlauf des Impulsdämpfungssignal (b) analog oder digital anzeigt, ausdruckt und/oder unter Vorgabe einer maximalen Dämpfung ein optisches und/oder akustisches Warnsignal abgibt.

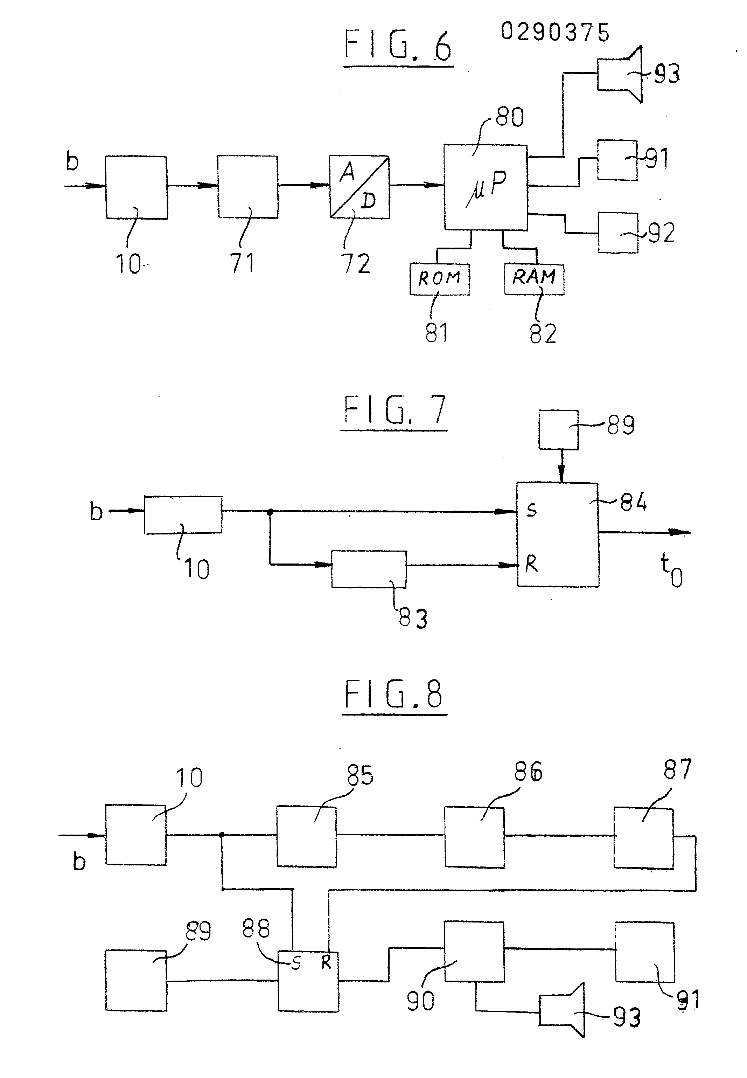

Das in Figur 6 dargestellte Blockschaltbild einer Ausführungsform der Signalspeicher- und Auswerteinrichtung gemäß Figur 5 zeigt ein die Impulsdämpfungssignale (b) empfangendes Filter 10, das nicht relevante Frequenzanteile des Impulsdämpfungssignals herausfiltert und das gefilterte Signal an den Eingang einer Einleseelektronik 71 anlegt, in der der Verlauf des Impulsdämpfungssignals (b) gespeichert wird. Anschließend wird das gespeicherte Signal mittels eines Analog/Digital-Wandlers 72 digitalisiert und mit einem Mikroprozessor 80 ausgewertet.

Der Mikroprozessor 80 ist mit einem Festspeicher bzw. ROM 81 sowie einem Speicher mit wahlfreiem Zugriff RAM 82 verbunden. Der Mikroprozessor 80 ermittelt aus dem digitalisierten Verlauf des Impulsdämpfungssignals (b) den Dämpfungsgrad nach einer oder mehreren der vorstehend beschriebenen Methoden und gibt ein Meßsignal als digitale oder analoge Ausgangsgröße an eine Anzeigevorrichtung 91 und/oder einen Drucker 92 ab. Zusätzlich kann durch Vorgabe einer maximalen zulässigen Dämpfung und Vergleich mit diesem maximalen Wert eine optische und/oder akustische Alarmeinrichtung 93 angesteuert werden, die dem Operateur das Erreichen des maximalen Dämpfungswertes unter Berücksichtigung eines Sicherheitsabstandes zu einem Dämpfungswert angibt, der mit großer Wahrscheinlichkeit zur Fraktur bzw. Fissur des schaftaufnehmenden Knochens führen würde.

Bei Verwendung eines Testgerätes 5 bzw. 6 gemäß den Figuren 2 oder 3 kann nach einer manuellen Auslösung vom Mikroprozessor 80 gesteuert eine Vielzahl von Anschlägen auf die Schaftprothese ausgelöst und aus den sich daraus ergebenden Impulsdämpfungssignalen ein Mittelwert gebildet werden, so daß Meßfehler oder Zufälligkeiten wie ein Einhaken der Prothese im schaftaufnehmenden Knochen eliminiert werden können. Selbstverständlich darf der vom Gerät 5, 6 ausgelöste Kraftimpuls einen bestimmten Wert nicht überschreiten, damit sichergestellt ist, daß die testweise Ermittlung der Festigkeit des Sitzes nicht zur befürchteten Fraktur bzw. Fissur führt.

In den Figuren 7 und 8 sind in vereinfachter Blockschaltbilddarstellung zwei Auswerteinrichtungen dargestellt, mit denen durch Auswertung des Impulsdämpfungssignals auf die Dämpfung und damit die Festigkeit des Sitzes zwischen Prothesenschaft und schaftaufnehmendem Knochen geschlossen werden kann.

Figur 7 zeigt das Blockschaltbild einer Vorrichtung zur Auswertung des Impulsdämpfungssignals (b) nach der oben dargestellten Methode a), wozu das Impulsdämpfungssignal (b) über einen Filter 10 an den Start bzw. Setzeingang eines Zählers 84 und über einen Nulldurchgangsdetektor 83 an den Stop bzw. Rücksetzeingang R des Zählers 84 gelegt wird. Der Zähler 84 ist mit einem Taktgeber 89 verbunden, so daß die Anzahl der Taktimpulse zwischen Start und Anhalten des Zählers 84 ein Maß für die Zeit zwischen dem Anschlagen der Prothese und dem ersten Nulldurchgang des Impulsdämpfungssignals ist, das in geeigneter Weise weiterverarbeitet und beispielsweise bei Erreichen eines minimalen Wertes zum Auslösen eines optischen und/oder akustischen Signals verwendet werden kann.

Figur 8 zeigt ein vereinfachtes Blockschaltbild einer Auswerteinrichtung, bei der der Nulldurchgang des Mittelwerts der gedämpften Schwingung des Impulsdämpfungssignals zur Auslösung eines optischen und/oder akustischen Signals verwendet wird.

Das Impulsdämpfungssignal (b) wird über ein Filter 10 sowohl an den Eingang eines Envelopendetektors 85 als auch an den Setz- bzw. Starteingang eines mit einem Taktgeber 89 verbundenen Zählers 88 gelegt. Der Ausgang des Envelopendetektors 85 ist mit einem Mittelwertbildner 86 verbunden, dessen Ausgang wiederum über einen Nulldurchgangsdetektor 87 an den Rücksetz- bzw. Stopeingang des Zählers 88 gelegt ist.

Das Ausgangssignal des Zählers 88, das der Zeitspanne zwischen dem Auftreffen der Einschlagvorrichtung auf die Schaftprothese und dem ersten Nulldurchgang des Mittelwerts der gedämpften Schwingung des Impulsdämpfungssignals entspricht, wird an einen Eingang eines Komparators 90 gelegt, dessen anderer Eingang mit dem Ausgang eines Grenzwertgebers 91 verbunden ist. Sind die an den beiden Eingängen des Komparators 90 anliegenden Signale gleich, so gibt der Komparator 90 ein Ausgangssignal an eine optische und/oder akustische Anzeigevorrichtung 92 ab, die unter Berücksichtigung eines Sicherheitsabstandes dem Operateur anzeigt, daß die optimale Festigkeit des Sitzes zwischen Schaftprothese und schaftaufnehmendem Knochen erreicht ist.

Die vorstehend nur beispielhaft beschriebenen Ausführungsbeispiele zur Ausgestaltung des Sensors bzw. Ausbildung der Signalspeicher- und Auswerteinrichtung können in vielfacher Weise modifiziert und kombiniert werden, ohne daß der erfindungsgemäße Gedanke, durch Ermittlung der Dämpfung bzw. Abbremsverzögerung der Prothese auf die Festigkeit des Sitzes zu schließen, verlassen wird.

Durch die meßtechnische Ermittlung der optimalen Festigkeit des Sitzes zwischen Schaftprothese und schaftaufnehmendem Knochen wird die intraoperative Sicherheit durch eine Standarisierung der Implantationstechnik bezüglich der Primärfestigkeit wesentlich erhöht und durch Einbeziehung eines ausreichenden Sicherheitsabstandes die Gefahr einer Fissur bzw. Fraktur des schaftaufnehmenden Knochens beseitigt bzw. vermindert.

Die Erfindung beschränkt sich in ihrer Ausführung nicht auf das vorstehend angegebene bevorzugte Ausführungsbeispiel. Vielmehr ist eine Anzahl von Varianten denkbar, welche von der dargestellten Lösung auch bei grundsätzlich anders gearteten Ausführungen Gebrauch machen. Insbesondere beschränkt sich die Ausführung nicht auf die Realisierung mit diskreten logischen Baugruppen, sondern läßt sich vorteilhaft auch mit programmierter Logik - vorzugsweise unter Verwendung eines Mikroprozessors - realisieren.

**Patentansprüche**

1. Vorrichtung zur meßtechnischen Kontrolle des Einschlagens einer zementfrei implantierbaren Schaftprothese in einen Knochenschaft mittels einer Einschlagvorrichtung, **gekennzeichnet durch** einen Sensor (4; 54, 64) zum Erfassen von Signalen im Knochen, die durch einen Schlag der Einschlagvorrichtung selbst oder einen entsprechenden Testgeber hervorgerufen sind, wobei der der Sensor (4; 54, 64) als Druck-, Bewegungs- oder Beschleunigungsempfänger ausgebildet ist, und eine Signalspeicher- und Auswerteinrichtung (7, 8), die ein vom Sensor (4; 54, 64) auf einen Schlag hin aufgenommenes Impulsdämpfungssignal (b) festhält, wobei die Auswertung unter Elimination der Amplitude des aufgenommenen Signals unabhängig von der Intensität des Einschlagimpulses erfolgt.

2. Vorrichtung nach Anspruch 1, **dadurch gekennzeichnet,** daß der Sensor (4) im Verlauf der Schlagachse eines Einschlaghammers (2) bzw. eines Einschlaginstruments (3) angeordnet ist.

3. Vorrichtung nach Anspruch 1, **dadurch gekennzeichnet,** daß der Sensor in einem Testgerät (5, 6) vorgesehen ist, das einen mechanisch oder elektromagnetisch angetriebenen Stößel (52, 62), eine Auslöseeinrichtung (63) und den die Beschleunigung bzw. Dämpfung des Stößels (52, 62) erfassenden Sensor (54, 64) aufweist.

4. Vorrichtung nach einem der vorstehenden Ansprüche, **dadurch gekennzeichnet,** daß der Sensor aus einem induktiven Aufnehmer (54) besteht.

5. Vorrichtung nach einem der vorstehenden Ansprüche 1 bis 3, **dadurch gekennzeichnet,** daß der Sensor aus einem piezokeramischen Wandler (64) besteht.

6. Vorrichtung nach Anspruch 1, **dadurch gekennzeichnet,** daß die Signalspeichereinrichtung (7) den zeitlichen Verlauf des Impulsdämpfungssignals (b), das der Beschleunigung bzw. Dämpfung oder Abbremsverzögerung der Schaftprothese (1) entspricht, registriert und daß die Auswerteinrichtung (8) den Dämpfungsgrad des Impulsdämpfungssignals (b) ermittelt und ein der Dämpfung proportionales Meßsignal an eine Anzeige-, Datenverarbeitungs- oder Druckeinrichtung (9) abgibt.

7. Vorrichtung nach Anspruch 6, **dadurch gekennzeichnet,** daß die Auswerteinrichtung (8) die Dämpfung des Impulsdämpfungssignals (b) mit einem vorgegebenen Maximalwert der Dämpfung vergleicht und bei einer Dämpfung, der größer oder gleich dem Maximalwert der Dämpfung ist, ein optisches und/oder akustisches Signal abgibt.

8. Vorrichtung nach einem der vorstehenden Ansprüche, **dadurch gekennzeichnet,** daß das Impulsdämpfungssignal (b) über ein Filter (10) an eine Einleseelektronik (71) zur Speicherung des Impulsdämpfungssignals abgegeben wird, deren Ausgang über einen Analog/Digital-Wandler (71) mit einem die Dämpfung bestimmenden Prozessor (80) verbunden ist, der ein der Dämpfung entsprechendes Signal an eine Anzeigeeinrichtung (91) und/oder einen Drukker (92) und/oder bei Überschreiten einer maximalen Dämpfung ein Freigabesignal an einen optischen und/oder akustischen Signalgeber (93) abgibt.

9. Vorrichtung nach einem vorstehenden Ansprüche 6 bis 8, **dadurch gekennzeichnet,** daß die Auswerteinrichtung (8) die Nulldurchgänge des Impulsdämpfungssignals (b) erfaßt und die Zeit ($t_0$) vom Aufschlag der Einschlag-

vorrichtung bis zum ersten Nulldurchgang des Impulsdämpfungssignals (b) und/oder die Zeit ($t_1$) von einem Nulldurchgang bis zu einem folgenden Nulldurchgang des Impulsdämpfungssignals (b) als Kriterium für dei Dämpfung verwendet.

10. Vorrichtung nach Anspruch 9, **dadurch gekennzeichnet,** daß das Impulsdämpfungssignal (b) sowohl an den Starteingang (S) eines mit einem Taktgenerator (89) verbundenen Zählers (84) als auch an den Eingang eines ersten Nulldurchgangsdetektors (83) gelegt ist, dessen Ausgang mit dem Stoppeingang (R) des Zählers (84) verbunden ist, an dessen Ausgang ein der Zeit ($t_0$) entsprechendes Signal ansteht.

11. Vorrichtung nach Anspruch 9, **dadurch gekennzeichnet,** daß das Impulsdämpfungssignal (b) über einen Filter (10) sowohl an den Eingang eines Envelopendetektors (85) als auch den Starteingang (S) eines Zählers (88) gelegt ist, daß der Ausgang des Envelopendetektors über einen Mittelwertbildner (86) mit dem Eingang eines Nulldurchgangsdetektors (87) verbunden ist, daß der Ausgang des Nulldurchgangsdetektors (87) mit dem Stoppeingang (R) des mit einem Taktgeber (89) verbundenen Zählers (88) verbunden ist, daß der Ausgang des Zählers (88) mit einem Eingang eines Komparators (90) verbunden ist, dessen anderer Eingang mit einem Grenzwertgeber (91) verbunden ist und daß der Ausgang des Komparators (90) an den Eingang eines Signalgebers (93) angeschlossen ist.

0290375

FIG.1

FIG.2

FIG.3

0290375

FIG. 4

FIG. 5

FIG. 6    0290375

FIG. 7

FIG. 8

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| A | DE-B-2 101 002  (AESCULAP) <br> * Spalte 4, Zeile 5 - Spalte 5, Zeile 11; Figuren 1,2 * <br> --- | 1 | A 61 F    2/46 <br> G 01 H   17/00 <br> B 25 D   13/00 <br> B 25 D    1/00 |
| A | DE-A-3 402 668  (STABILATOR) <br> * Zusammenfassung; Figuren 2,4 * <br> --- | 1-3 | |
| A | DE-A-3 115 711  (FRAUNHOFER) <br> * Seite 4, Zeile 21 - Seite 5, Zeile 30; Figur * <br> --- | 1-4,6 | |
| A | DE-A-1 913 190  (HUGGLER) <br> * Seite 4, Zeile 3 - Seite 6, Zeile 14; Figuren * <br> --- | 1 | |
| A | EP-A-0 093 896  (SIEMENS) <br> * Seite 5, Zeile 4 - Seite 10, Zeile 6; Figuren * <br> --- | 1-3,5-7 ,9-11 | |
| A | DE-A-3 028 187  (BOSCH) <br> * Seite 4, Zeile 17 - Seite 5, Zeile 17; Zusammenfassung; Figuren * <br> --- | 2-5 | |
| A | DE-A-1 933 440  (C.P.K.T. B.V.) <br> --- | | RECHERCHIERTE SACHGEBIETE (Int. Cl.4) |
| A | US-A-4 318 302  (CHOI) <br> --- | | A 61 F <br> A 61 B <br> G 01 H <br> G 01 V <br> B 25 D <br> A 61 C |
| A | EP-A-0 181 131  (K.S.I.P.L.) <br> ----- | | |

Der vorliegende Recherchenbericht wurde für alle Patentansprüche erstellt

| Recherchenort | Abschlußdatum der Recherche | Prüfer |
|---|---|---|
| DEN HAAG | 16-08-1988 | KLEIN C. |